# EUROPEAN PATENT SPECIFICATION

(11) **EP 3 582 787 B1**
(45) Date of publication and mention of the grant of the patent: **10.09.2025**
(21) Application number: 18754040.6
(22) Date of filing: 13.02.2018
(51) Int. Cl.: A61K 31/7016, A61K 33/00, A61K 35/28, A61P 25/28, A61P 29/00, A61P 27/02, A61K 45/06, A61K 9/00

(54) **PHARMACEUTICAL PREPARATION FOR THE PREVENTION AND TREATMENT OF INFLAMMATORY AND DEGENERATIVE DISEASES**
PHARMAZEUTISCHE ZUBEREITUNG ZUR PRÄVENTION UND BEHANDLUNG VON ENTZÜNDUNGS- UND DEGENERATIVEN ERKRANKUNGEN
PRÉPARATION PHARMACEUTIQUE POUR LA PRÉVENTION ET LE TRAITEMENT DE MALADIES INFLAMMATOIRES ET DÉGÉNÉRATIVES

(30) Priority: 14.02.2017 CZ 20170084
(43) Date of publication of application: 25.12.2019
(73) Proprietor: Ustav Experimentalni Mediciny Akademie Ved Cr, v.v.i., 14220 Praha (CZ)
(72) Inventor: CEJKOVA, Jitka, 102 00 Praha 10 (CZ); CEJKA, Cestmir, 120 00 Praha 2 (CZ); KUBINOVA, Sarka, 19000 Praha 9 (CZ); VACKOVA, Irena, 104 00 Praha 22 (CZ)
(74) Representative: Havlík, Michal
(86) International application number: PCT/CZ2018/000009
(87) International publication number: WO 2018/149426

(56) References cited:
- EP-A1- 1 946 762
- WO-A1-2009/104822
- WO-A1-2016/130478
- WO-A2-2014/026117
- CZ-U1- 30 239
- MIYUKI KUBOTA ET AL: "Hydrogen and N-Acetyl-L-Cysteine Rescue Oxidative Stress-Induced Angiogenesis in a Mouse Corneal Alkali-Burn Model", INVESTIGATIVE OPHTHALMOLOGY & VISUAL SCIENCE, vol. 52, no. 1, 21 January 2011 (2011-01-21), pages 427 - 433, XP055535711
- JITKA CEJKOVÁ ET AL: "Favorable effects of trehalose on the development of UVB-mediated antioxidant/pro-oxidant imbalance in the corneal epithelium, proinflammatory cytokine and matrix metalloproteinase induction, and heat shock protein 70 expression", GRAEFE'S ARCHIVE FOR CLINICAL AND EXPERIMENTAL OPHTHALMOLOGY ; INCORPORATING GERMAN JOURNAL OF OPHTHALMOLOGY, SPRINGER, BERLIN, DE, vol. 249, no. 8, 15 April 2011 (2011-04-15), pages 1185 - 1194, XP019935439, ISSN: 1435-702X, DOI: 10.1007/S00417-011-1676-Y
- CESTMIR CEJKA ET AL: "Oxidative Stress to the Cornea, Changes in Corneal Optical Properties, and Advances in Treatment of Corneal Oxidative Injuries", OXIDATIVE MEDICINE AND CELLULAR LONGEVITY, vol. 2015, 1 January 2015 (2015-01-01), US, pages 1 - 10, XP055535630, ISSN: 1942-0900, DOI: 10.1155/2015/591530
- CEJKOVA JITKA ET AL: "Suppression of alkali-induced oxidative injury in the cornea by mesenchymal stem cells growing on nanofiber scaffolds and transferred onto the damaged corneal surface", EXPERIMENTAL EYE RESEARCH, ACADEMIC PRESS LTD, LONDON, vol. 116, 18 October 2013 (2013-10-18), pages 312 - 323, XP028779731, ISSN: 0014-4835, DOI: 10.1016/J.EXER.2013.10.002
- CEJKA CESTMIR ET AL.: "Oxidative Stress to the Cornea, Changes in Corneal Properties, and Advances in Treatment of Corneal Injuries", OXIDATIVE MEDICINE AND CELLULAR LONGEVITY 2015, vol. 2015, 11 March 2015 (2015-03-11), pages 1 - 10, XP055535630, ISSN: 1942-0900
- CEJKOVA JITKA ET AL.: "The role of oxidative stress in corneal disease and injuries", HISTOLOGY AND HISTOPATHOLOGY, vol. 30, 24 March 2015 (2015-03-24), pages 893 - 900, XP055535701, ISSN: 0213-3911
- OHTA SHIGEO: "Recent Progress Toward Hydrogen Medicine: Potential of Molecular Hydrogen for Preventive and Therapeutic Applications", CURRENT PHARMACEUTICAL DESIGN, vol. 17, no. 22, 1 July 2011 (2011-07-01), pages 2241 - 2252, XP055535704, ISSN: 1381-6128
- KUBOTA MIYUKI ET AL.: "Hydrogen and N-acetyl-L-Cysteine Rescue Oxidative Stress-Induced Angiogenesis in a Mouse Corneal Alkali-Burn Model", INVESTIGATIVE OPHTHALMOLOGY & VISUAL SCIENCE, vol. 52, no. 1, 21 January 2011 (2011-01-21), pages 427 - 433, XP055535711, ISSN: 0146-0404
- HONG Y. ET AL.: "Hydrogen as a Selective Antioxidant: a Review of Clinical and Experimental Studies", THE JOURNAL OF INTERNATIONAL MEDICAL RESEARCH, vol. 38, 1 December 2010 (2010-12-01), pages 1893 - 1903, XP055535712, ISSN: 0300-0605
- YOKOTA TAKASHI ET AL.: "Protective effect of molecular hydrogen against oxidative stress caused by peroxynitrite derived from nitric oxide in rat retina", CLINICAL & EXPERIMENTAL OPHTHALMOLOGY, vol. 43, no. 6, 3 May 2015 (2015-05-03), pages 568 - 577, XP055535723, ISSN: 1442-6404
- SUN JING-CHUAN ET AL.: "Hydrogen-Rich Saline Promotes Survival of Retinal Ganglion Cells in a Rat Model of Optic Nerve Crush", PLOS ONE, vol. 9, no. 6, 10 June 2014 (2014-06-10), pages e99299, XP055535726, ISSN: 1932-6203

## Description

### Field of the invention

The invention relates to a pharmaceutical preparation comprising molecular hydrogen H₂, with enhanced antioxidant effect, intended for use in the treatment of an inflammatory or degenerative disease of the cornea and/or retina.

### Background of the invention

Oxidative stress causes and promotes inflammatory diseases and plays an important role in cancer and degenerative diseases as well as in ageing. Oxidative stress results from an imbalance between the systemic manifestation of reactive oxygen species (ROS) and the capacity of anti-oxidation systems where ROS are not sufficiently decomposed, resulting in oxidative damage to the cells due to peroxidation of lipids, oxidative protein changes and oxidative damage to DNA. Oxidative stress plays a negative role in each and every branch of medicine. In ophthalmology, for example, oxidative stress triggers and promotes inflammation of the cornea, causes many types of cataract, uveitis, retinopathy in premature babies, age-related macular degeneration (AMD) and some kinds of glaucoma.

Oxidative stress plays a particular role in the ischemia-reperfusion injury. The ischemia-reperfusion injury is the cause of acute oxidative stress with critical consequences. Such disorder occurs when tissues are affected by a sudden lack of oxygen (so-called ischemia) followed by a restoration of oxygen supply (so-called reperfusion). It occurs e.g. in relation to myocardial infarction, diseases of kidneys and liver, cornea and retina as well as other organs, such as, for example, in spine and spinal cord injuries associated with movement disorders. The administration of high doses of antioxidants or anti-inflammatory medicaments often fails to achieve a convincing effect, and in many cases have even been experienced serious adverse side effects.

Certain studies have suggested the therapeutic application of molecular hydrogen H₂ based on its anti-inflammatory effect. Small molecules of molecular hydrogen H₂ penetrate the damaged tissues and cells and quickly reach the nucleus. In the nucleus, they decompose reactive oxy-radicals.

Patent US 9050278 describes a preparation for capturing harmful reactive oxygen species consisting of a solution comprising molecular hydrogen H₂. The patent teaches the application in the treatment of ischemia-reperfusion injury of tissue.

Patent application US 2007148256 discloses a pharmaceutical preparation on the basis of a solution comprising molecular hydrogen and colloid precious metal particles which catalyze the disintegration of molecular hydrogen H₂ to atomic hydrogen H.

WO 2014/026117 discloses neuroprotective liposome compositions and methods for the treatment of stroke. In one embodiment compositions for use in treating stroke, such as liposomes loaded with Xe or Xe in combination with H₂ or H₂S are disclosed.

Kubota et al., 2011, Invest Opht Vis Sci, 52(1), 427-433 identifies immediate antioxidant therapy with H₂-enriched irrigation solution as a new potent treatment of angiogenesis in cornea to prevent blindness caused by alkali burn.

Čejková et al, 2011, Graefe's Arch Clin Exp Opht, 249 (8), 1185-1194 reported that trehalose favorably influenced the oxidative damage of the cornea caused by UVB rays. Trehalose suppressed proinflammatory cytokine induction. It was suggested that suppression of proinflammatory cytokines contributed strongly to reduced matrix metalloproteinase and xanthine oxidase expression in the UVB-irradiated corneal epithelium and to the decreased development of an antioxidant/pro-oxidant imbalance. The overexpression of heat shock protein 70 found in UVB-irradiated cornea after buffered saline treatment was reduced after trehalose application.

Čejka et al, 2015, Ox Med Cell Long, 1-10 described that oxidative stress in the cornea appearing after the influence of various environmental noxae may be involved in the initiation as well as propagation of corneal disturbances accompanied by changes in corneal optical properties and decrease in visual acuity or even vision loss. The paper concluded that the drugs with antioxidant effects suppressing the oxidative injury are necessary for corneal healing and renewal of corneal optical properties inevitable for vision restoration.

Čejková et al, 2013, Exp Eye Res, 116, 312-323 reported that mesenchymal stem cells effectively reduce alkali-induced oxidative stress in the cornea, leading to the suppression of intracorneal inflammation as well as a decrease in corneal neovascularization, resulting in significantly accelerated healing of the corneas.

Human medicine uses trehalose abundantly, for instance to prevent water loss in cells (dehydration), prevent cell exposure to excess heat, cold or oxy-radicals, against hypoxia to anoxia and against hypoxia/re-oxygenation injury.

For instance, patent EP 580 444 discloses the use of trehalose solution to keep organs for transplantation.

Patent application EP 2 848 683 A1 teaches the addition of trehalose to the suspension when stem cell suspension is administered via the vascular system.

### Summary of the invention

The invention is based on the idea of using molecular hydrogen H₂ in combination with another anti-inflammatory and anti-oxidative agent. As has been found by experiments, the anti-oxidative effects of molecular hydrogen H₂ increase in synergic way in the presence of trehalose.

Therefore, the essence of the invention consists of combining molecular hydrogen H₂ with trehalose, which is an efficient anti-stress, anti-inflammatory and anti-oxidative factor. The combination of molecular hydrogen H₂ and trehalose, particularly in the form of preparations containing molecular hydrogen H₂ and trehalose, may be used in the prevention and treatment of a broad spectrum of inflammatory and degenerative diseases.

Thus, the present invention relates to the pharmaceutical preparation for use in the treatment of an inflammatory or degenerative disease of the cornea and/or retina containing 0.15 to 0.8 mmol of hydrogen H₂ and 0.5 to 500 mmol of trehalose per litre of pharmaceutical solution, preferably 5 to 300 mmol of trehalose per litre of pharmaceutical solution. The aqueous basis of the pharmaceutical solution may be saline solution, PBS (phosphate buffered saline), water for injection (aqua pro injectione) and/or other pharmacologically acceptable medium. The pharmaceutical preparation according to the invention may also be prepared in the form of an aqueous solution of trehalose enriched with molecular hydrogen H₂ for oral administration.

Although the preferred pharmaceutical preparation according to the invention comprises 0.15 to 0.8 mmol of hydrogen H₂ and, for some applications, it is most preferred in the form of a solution saturated or almost saturated with molecular hydrogen H₂ which contains approximately 0.6 to 0.8 mmol H2 per litre, it is to be noted that within the scope of the invention, hydrogen H₂ and trehalose may be administered separately. Therefore, the invention encompasses a combination of molecular hydrogen H₂ with trehalose for the prevention and treatment of inflammatory and degenerative diseases where trehalose may, but does not have to, be applied in the form of a solution comprising dissolved molecular hydrogen H₂. Trehalose may be combined with hydrogen H₂ even in a manner permitting inhalation of hydrogen H₂, or where hydrogen H₂ is a part of medicinal gas used for anaesthetic purposes (general anaesthesia). Trehalose is then administered topically, orally or injected.

Of course the pharmaceutical preparation according to the invention may be applied in conjunction with another suitable treatment method. It has been found out that the anti-oxidative effects of molecular hydrogen H₂ and trehalose may contribute particularly towards the treatment of mesenchymal stem cell (MSC) application.

The pharmaceutical preparation according to the following embodiment of the invention contains a suspension of mesenchymal stem cells in a pharmaceutical solution comprising 0.15 to 0.8 mmol of molecular hydrogen H₂ and 0.5 to 500 mmol trehalose per litre of pharmaceutical solution, preferably 5 to 300 mmol trehalose per litre of pharmaceutical solution and, most preferably in this embodiment of the invention, 10 to 300 mmol trehalose per litre of pharmaceutical solution.

As has been mentioned above, the pharmaceutical preparation according to the invention contains trehalose, most preferably in a pharmaceutical solution saturated or almost saturated with molecular hydrogen H₂. The saturation level corresponds to a content of molecular hydrogen H₂ amounting to approximately 0.8 mmol per litre of pharmaceutical solution.

The synergic effect of the preparation according to the invention in combination with stem cells must also be attributed to the fact that the pharmaceutical preparation has an anti-oxidative and antiallergenic effect on the stem cells, and regulates cell genes; this enhances the regenerative effect of stem cells on the tissue.

As has been mentioned above, the pharmaceutical preparation according to the invention is intended for the prevention and treatment of inflammatory and degenerative diseases. However, it is particularly effective if used to treat inflammatory and degenerative diseases of both anterior and posterior segment of the eye (particularly cornea and retina).

Therefore, the invention specifically encompasses the pharmaceutical preparation for the use as defined in claim1 which contains 0.15 to 0.8 mmol of molecular hydrogen H₂, most preferably 0.6 to 0.8 mmol of molecular hydrogen H₂, and 0.5 to 500 mmol trehalose, preferably 5 to 300 mmol trehalose, most preferably 10 to 300 mmol trehalose per litre of pharmaceutical solution. The pharmaceutical preparation preferably consists of pharmaceutical solution saturated with molecular hydrogen H₂.

Although not claimed, the description also relates to the use of the preparation according to the invention in combination with stem cells to treat inflammatory and degenerative diseases of the cornea.

In a specific implementation, the stem cells may be preferably used with a nanofiber carrier. Another embodiment of this disclosure, not part of the claimed invention, consists of the use of the preparation according to the invention as supporting medication in the treatment of inflammatory and degenerative diseases of the cornea by means of mesenchymal stem cells applied to or incubated on nanofibers.

The new approach to oxidative stress reduction and thus preventing tissue damage according to the invention is based on the effect of the small molecule of molecular hydrogen H₂ which allows rapid penetration through the damaged tissues and cells. Hydrogen H₂ penetrates the cells and reaches the mitochondria and the cell nucleus while decomposing hydroxyl radicals and peroxynitrite formed by the reaction between superoxide and nitric oxide. Hydrogen H₂ is capable of crossing the hematoencephalic barrier which separates the internal brain environment from the vascular system. Similarly, hydrogen H₂ - in contrast to other antioxidants - crosses the hemato-ocular barrier.

Hydrogen H₂ does not react with cells and tissues at all, even at high concentrations. In combination with trehalose which is an efficient anti-stress, anti-inflammatory and anti-oxidative factor, as well as being absolutely non-toxic even at high concentrations, it allows optimal effect on the cells and tissues. Upon hypoxia, trehalose protects cells from damage by preventing protein degradation and stabilises cellular membranes. Trehalose also has anti-inflammatory and anti-oxidative effects. It influences cellular gene expression. This is particularly important in some neurodegenerative diseases where the cellular genes are damaged.

The ultimate effects of hydrogen H₂ and trehalose are the prevention or efficient suppression of oxidative stress, reduction of inflammation and cellular apoptosis, which is followed by rapid tissue regeneration and accelerated healing. With hypoxia-reperfusion injury, the combination of hydrogen H₂ and trehalose has a prophylactic as well as therapeutic effect in both acute and chronic stages. If the combination of hydrogen H₂ with trehalose is administered in due time in case of lesions caused by hypoxia, hydrogen H₂ prevents ROS from forming in the course of the subsequent reperfusion.

The combination of hydrogen H₂ with trehalose minimises the occurrence of scar tissue. This effect is of special significance in the cornea where the formation of a scar means a loss of cornea transparency. In cases where the combination of hydrogen H₂ with trehalose in the solution is accompanied by stem cells, mesenchymal stem cells (MSCs) in particular, the efficacy of such pharmaceutical preparation is potentiated by their anti-oxidative, anti-inflammatory and regenerative activity. References to methods of treatment in the subsequent paragraphs of this description are to be interpreted as references to the pharmaceutical preparations of the present invention for use in a method for treatment of the human body by therapy.

### Examples

### Example 1: Reducing oxidative stress upon chemical burn of the eye using the preparation according to the invention

Rabbit eyes were experimentally burnt by 0.25N solution of NaOH (applying 10 drops to the eye over 1 minute; the rabbits were under general anaesthesia) and the rabbits were divided into five groups of samples. Immediately after the burn, eyes of some of the rabbits were repeatedly rinsed with a therapeutic preparation formed by saturation of 280mM of trehalose solution in PBS with hydrogen H₂ (the H₂ content corresponding with the saturation state was approximately 0.8 mmol per litre). The saturation of the pharmaceutical solution by molecular hydrogen, once the trehalose has dissolved in PBS, was performed by applying a commercially available chemical cartridge of Dr. H. Hayashi's original HydrogenRich Water Stick^{™} or by driving hydrogen gas through the solution. The level of saturation may be detected by a probe for molecular hydrogen concentration measurement in the solution or, indirectly, by means of its oxidation-reduction potential; the state of saturation corresponds to the value of -600 mV. The burnt eyes were rinsed over and over again for the next five days (five times a day). The eyes of the second group of samples were rinsed in a similar manner, with PBS alone saturated with hydrogen H₂; for the third sample, only PBS with trehalose was used while the fourth sample had eyes rinsed merely with PBS after burning. The fifth part of the rabbits formed a sample that received no treatment whatsoever. The central corneal thickness was measured by ultrasonic pachymeter (once a day), and photographic records were made of the eyes. The experiment ended on the 20^{th} day from the day of the chemical burns. The rabbits were killed under general anaesthesia and their eyes were biochemically and immunohistochemically examined. The results demonstrated that the burnt eyes treated by PBS, identically to untreated eyes, were seriously damaged by oxidative stress which occurred immediately after the burning. Oxidative stress was detected directly by the analysis of reactive oxygen species (ROS) in the tissues by dihydroethidium and indirectly by the imbalance between the expression and activity of anti-oxidative and pro-oxidative enzymes in the corneal epithelium. The transparency of the eyes was lost. Both immunohistochemical and biochemical examinations were performed to determine pro-inflammatory cytokines, peroxynitrite and malondialdehyde, oxidative stress and lipid peroxidation markers. The corneas treated by trehalose or hydrogen H₂ showed suppression of pro-inflammatory cytokines, peroxynitrite and malondialdehyde.

However, the best results were obtained when the pharmaceutical preparation containing a combination of hydrogen H₂ with trehalose was applied. This pharmaceutical preparation prevented oxidative stress and its consequences in the cornea. The corneas healed with restored transparency. The optical properties of the anterior segment of the eye were restored.

Indicative experiments discovered that the pharmaceutical preparation according to the invention delivers the best results with a concentration of 5 or 10 to 300 mmol trehalose per litre of pharmaceutical solution and with a content of molecular hydrogen H₂ from 0.6 mmol per litre of such pharmaceutical solution to its saturation by molecular hydrogen H₂.

Unless otherwise indicated, the following examples used a 280mM solution of trehalose in PBS, saturated with hydrogen H₂ at room temperature as the pharmaceutical preparation according to the invention.

### Example 2: Suppressing oxidative stress upon ultraviolet (UV) irradiation of the eye by using the preparation according to the invention

Rabbit eyes were repeatedly irradiated by UBV (i.e. medium-length UV radiation) with energy of 1.01 J/cm², once a day for four days. After each irradiation, the eyes were rinsed with PBS saturated with hydrogen H₂ and containing trehalose (concentration of 280 mmol per litre) for five minutes. In the course of irradiation, this group of rabbits was continuously compared (photographic evidence was taken of their eyes and the corneal thickness was measured by ultrasonic pachymeter) to a group of rabbits whose eyes were not rinsed with the pharmaceutical preparation. After the fourth irradiation, the corneas in this group were non-transparent, with an off-white haze, hydrated with starting neovascularisation in the limbal ring. In contrast to that, in the eyes that had been irradiated and then rinsed with PBS with trehalose and hydrogen H₂, the corneas were transparent, just slightly opalescent, with no neovascularisation and increased corneal hydration as well as no increased corneal thickness.

### Example 3: Treatment of eyes damaged by UV rays using the preparation according to the invention

Rabbit eyes were repeatedly irradiated with UBV rays (1.01 J/cm2, once per day, over 4 days). Afterwards, the rabbits were divided into four groups. In the first group, the eyes were treated with a preparation containing PBS saturated with hydrogen H₂ and containing trehalose at a concentration of 500 mmol per litre. In the second group, the eyes were treated with PBS saturated with hydrogen H₂, in the third group the treatment involved PBS with trehalose and the fourth group had eyes treated merely by PBS. The treatment consisted of the application of the relevant solutions to the surface of the eye, five times per day over three weeks. Then the rabbits from all groups were killed under general anaesthesia and their eyes were examined biochemically and by microscope. The results showed that rabbit eyes with non-transparent thickened corneas after repetitive UVB irradiation healed best, with transparency restoration, when treated with the preparation containing trehalose and hydrogen H₂. The preparation containing just hydrogen H₂ or the preparation containing solely trehalose caused a partial restoration of corneal transparency. The corneas healed by PBS alone retained the off-white cloudiness and vascularisation at the end of the experiment.

### Example 4: Treating eyes damaged by UV rays with mesenchymal stem cells using the preparation according to the invention

Rabbit eyes were repetitively irradiated by UBV rays (1.01 J/cm², once per day over four days). After each irradiation, the eyes were rinsed for 5 minutes with PBS saturated with hydrogen H₂. Once the irradiation period was finished, nanofibers with MSC (in this case, the MSCs were stem cells from rabbit bone marrow which had been incubated on the nanofibers in advance) were stitched onto the eye. PBS with trehalose and hydrogen H₂ was applied on top of the nanofiber with MSCs (20 drops over 2 minutes); the concentration of hydrogen H₂ was 0.8 mmol per litre and the concentration of trehalose was 280 mmol per litre. Then, the eye was stitched closed and left with no further treatment for 5 days. After these five days, the stitches were taken out and the nanofibers removed. Then, the eye was left with no treatment for 3 weeks. The healing of the eye was monitored in terms of macroscopic, photographic evidence and measurement of central corneal thickness using ultrasonic pachymeter. After three weeks, the rabbits were killed and their eyes underwent microscopic and biochemical examinations. The test group of rabbits that received the treatment was compared to other groups of rabbits with cornea exposed to the same radiation, yet not receiving any treatment or only treated with nanofibers, or with a solution of PBS with trehalose. In these groups, the test rabbits were also killed three weeks after the damage. The results proved that untreated eyes were ulcerated, the eyes treated with the pharmaceutical preparation with trehalose and hydrogen H₂ healed with no ulcers and without non-transparent scars, and corneal transparency was restored. The eyes treated with MSCs transferred via nanofibers with the pharmaceutical preparation with trehalose and hydrogen H₂ healed most quickly and with the least residual changes in transparency.

### Example 5: Treatment of glaucoma using the preparation according to the invention

In the case of glaucoma, higher intraocular pressure causes reduced thickness of the retina through the ischemia-reperfusion injury mechanism and the subsequent effect of ROS. When drops of saline solution saturated with H₂ and containing 280 mmol per litre trehalose were administered (dripped on the surface of the eyes) in the rabbit model, the cellular damage decreased and cellular apoptosis, occurrence of malondialdehyde and peroxinitride, oxidative stress markers and lipid peroxidation were reduced. Control drops of saline solution yielded no positive results. Saline solution with H₂ alone or trehalose alone delivered a partial positive result: oxidative stress suppression. The best results were observed in the case of drops containing the combination of H₂ with trehalose.

### Example 6: Treating retina damaged by ischemia-reperfusion injury using the preparation according to the invention

Damage to retina caused by hypoxia-reperfusion injury with increased intraocular pressure causes neuronal problems due to ROS. An experimentally induced retinal ischemia due to increased intraocular pressure caused ROS generation and retinal cell apoptosis already in 24 hours. Retinal thickness measurement discovered a reduction of the thickness in the course of several days following the ischemia. The pharmaceutical preparation applied to the surface of the eye reduced the number of apoptotic retinal cells considerably, reduced ROS and prevented retinal thickness reduction. The pharmaceutical preparation according to the invention proved an anti-oxidative and neuroprotective effect.

Increased effect of the application of the pharmaceutical preparation according to the invention on the surface of the eyes was observed in experiments wherein the test animals were given, in the form of drink, 3%-wt (88 mmol per litre) solution of trehalose in water saturated with molecular hydrogen H₂.

### Example 7: Treatment of spinal cord damaged by a trauma to the spine using the preparation according to the invention. Example not part of the claimed invention but useful for its understanding.

Damage to the spinal cord after a spinal injury results in the activation of astrocytes, caused by oxidative stress following a hypoxia-reperfusion injury accompanying a spinal trauma. Reactive astrogliosis is accompanied by excessive formation of pro-inflammatory cytokines in the astrocytes, and release thereof to the surroundings. This potentiates the inflammation. The pharmaceutical preparation according to the invention, intravenously injected, reduced or completely prevented any excessive inflammatory process at the trauma spot, and greatly improved post-traumatic locomotion.

### Example 8: Treatment of inflammatory diseases using the preparation according to the invention Example not part of the claimed invention but useful for its understanding.

Indicative experiments have shown that the pharmaceutical preparation according to the invention contributes towards the treatment of inflammatory diseases, including post-traumatic inflammatory response to damage or injury. The pharmaceutical preparation according to the invention, if injected intravenously, reduced or completely prevented excessive inflammatory processes at the damage spot.

Similar results were obtained from animal models in relation to inflammatory diseases of connective tissues, primarily diseases of the joints and mouth (like periodontium, the connective mechanism of teeth).

Drinking trehalose solution in water saturated with molecular hydrogen H₂ was observed to boost the effect of treatment.

## Claims

1. A pharmaceutical preparation for use in the treatment of an inflammatory or degenerative disease of the cornea and/or retina, **characterised in that** it comprises molecular hydrogen H₂ and trehalose in a solution comprising 0.15 to 0.8 mmol of molecular hydrogen H₂ per litre of solution, and 0.5 to 500 mmol trehalose per litre of solution.

2. The pharmaceutical preparation according to claim 1 **characterised in that** it comprises molecular hydrogen H₂ and trehalose in a pharmaceutical solution comprising 5 to 300 mmol, preferably 10 to 300 mmol trehalose per litre of solution.

3. The pharmaceutical preparation according to claim 1 or 2 **characterised in that** it comprises molecular hydrogen H₂ and trehalose in a pharmaceutical solution comprising 0.6 to 0.8 mmol of molecular hydrogen H₂ per litre of pharmaceutical solution, preferably in a pharmaceutical solution saturated with molecular hydrogen H₂.

4. The pharmaceutical preparation according to any one of the preceding claims **characterised in that** the basis of the pharmaceutical solution is saline solution or PBS or water for injection.

5. The pharmaceutical preparation according to any one of the preceding claims, **characterised in that** it also comprises stem cells, preferably mesenchymal stem cells.

6. The pharmaceutical preparation according to claim 5, **characterised in that** it comprises a suspension of stem cells in the pharmaceutical solution containing molecular hydrogen H₂ and trehalose.

## Patentansprüche

1. Pharmazeutische Zubereitung zur Verwendung bei der Behandlung einer entzündlichen oder degenerativen Erkrankung der Hornhaut und/oder der Netzhaut, **dadurch gekennzeichnet, dass** sie molekularen Wasserstoff H₂ und Trehalose in einer Lösung enthält, die 0,15 bis 0,8 mmol molekularen Wasserstoff H₂ pro Liter Lösung und 0,5 bis 500 mmol Trehalose pro Liter Lösung enthält.

2. Pharmazeutische Zubereitung nach Anspruch 1, **dadurch gekennzeichnet, dass** sie molekularen Wasserstoff H₂ und Trehalose in einer pharmazeutischen Lösung enthält, die 5 bis 300 mmol, vorzugsweise 10 bis 300 mmol Trehalose pro Liter Lösung enthält.

3. Pharmazeutische Zubereitung nach Anspruch 1 oder 2, **dadurch gekennzeichnet, dass** sie molekularen Wasserstoff H₂ und Trehalose in einer pharmazeutischen Lösung umfasst, die 0,6 bis 0,8 mmol molekularen Wasserstoff H₂ pro Liter pharmazeutischer Lösung, vorzugsweise in einer mit molekularem Wasserstoff H₂ gesättigten pharmazeutischen Lösung, umfasst.

4. Die pharmazeutische Zubereitung nach einem der vorstehenden Ansprüche, **dadurch gekennzeichnet, dass** die pharmazeutische Lösung auf Kochsalzlösung oder PBS oder Wasser für Injektionszwecke basiert.

5. Pharmazeutische Zubereitung nach einem der vorstehenden Ansprüche, **dadurch gekennzeichnet, dass** sie auch Stammzellen, vorzugsweise mesenchymale Stammzellen, umfasst.

6. Pharmazeutische Zubereitung nach Anspruch 5, **dadurch gekennzeichnet, dass** sie eine Suspension von Stammzellen in der pharmazeutischen Lösung enthält, die molekularen Wasserstoff H₂ und Trehalose enthält.

## Revendications

1. Préparation pharmaceutique destinée à être utilisée dans le traitement d'une maladie inflammatoire ou dégénérative de la cornée et/ou de la rétine, **caractérisée en ce qu'**elle comprend de l'hydrogène moléculaire H₂ et du tréhalose dans une solution comprenant 0,15 à 0,8 mmol d'hydrogène moléculaire H₂ par litre de solution, et 0,5 à 500 mmol de tréhalose par litre de solution.

2. La préparation pharmaceutique selon la revendication 1, **caractérisée en ce qu'**elle comprend de l'hydrogène moléculaire H₂ et du tréhalose dans une solution pharmaceutique comprenant 5 à 300 mmol, de préférence 10 à 300 mmol de tréhalose par litre de solution.

3. La préparation pharmaceutique selon la revendication 1 ou 2, **caractérisée en ce qu'**elle comprend de l'hydrogène moléculaire H₂ et du tréhalose dans une solution pharmaceutique comprenant 0,6 à 0,8 mmol d'hydrogène moléculaire H₂ par litre de solution pharmaceutique, de préférence dans une solution pharmaceutique saturée en hydrogène moléculaire H₂.

4. La préparation pharmaceutique selon l'une quelconque des revendications précédentes, **caractérisée en ce que** la base de la solution pharmaceutique est une solution saline ou du PBS ou de l'eau pour injection.

5. La préparation pharmaceutique selon l'une quelconque des revendications précédentes, **caractérisée en ce qu'**elle comprend également des cellules souches, de préférence des cellules souches mésenchymateuses.

6. La préparation pharmaceutique selon la revendication 5, **caractérisée en ce qu'**elle comprend une suspension de cellules souches dans la solution pharmaceutique contenant de l'hydrogène moléculaire H₂ et du tréhalose.
